# EUROPÄISCHE PATENTANMELDUNG

(11) **EP 1 878 457 A2**
(43) Veröffentlichungstag der Anmeldung: **16.01.2008**
(21) Anmeldenummer: 07011635.5
(22) Anmeldetag: 14.06.2007
(51) Int. Cl.: A61M 16/00

(54) **Beatmungsgerät zur Behandlung von obstruktiver Schlafapnoe und Verfahren zu dessen Steuerung**

(30) Priorität: 13.07.2006 DE 102006032620
(71) Anmelder: Hoffrichter GmbH, 19061 Schwerin (DE)
(72) Erfinder: Hoffrichter, Helmut, 19075 Pampow (DE)
(74) Vertreter: von Eichel-Streiber, Caspar

(57) **Zusammenfassung**

Beschrieben wird ein Beatmungsgerät (1) zur Behandlung von obstruktiver Schlafapnoe, mit einem Druckerzeuger (3) zum Erzeugen einer künstlichen Atmosphäre mit einem Therapiedruck (p_{act}), mit einer Druckregeleinrichtung (10) zum Führen des Druckerzeugers (3), und mit einem Atemschlauch (6) mit Atemmaske (7) zum Zuführen der von dem Druckerzeuger (3) erzeugten künstlichen Atmosphäre an einen Patienten (2), welches sich dadurch auszeichnet, dass es eine Steuerung (13) zum kontinuierlichen Ermitteln eines über einem Atemwegswiderstand (R_{AW}) der Atemwege des Patienten anfallenden Verlustdruckes (Δp) und zur im wesentlichen proportionalen Anpassung des Therapiedruckverlaufes an den Verlauf des ermittelten Verlustdruckes (Δp) aufweist.

Ein solches Beatmungsgerät zur Therapie von Schlafapnoe anzugeben reduziert in der Anwendung die Belastung der Atemmuskulatur des Patienten erheblich, macht den Behandlungsverlauf angenehmer und verringert eventuelle Spätfolgen der Behandlung.

## Beschreibung

Die Erfindung betrifft ein Beatmungsgerät zur Behandlung von Patienten, die an obstruktiver Schlafapnoe leiden, mit den Merkmalen des Oberbegriffes des Anspruchs 1 und ein Verfahren zu dessen Steuerung.

Beatmungsgeräte zur Behandlung der obstruktiven Schlafapnoe erzeugen eine künstliche Atmosphäre, die relativ zur natürlichen Atmosphäre einen höheren Druck aufweist und dem zu behandelnden Patienten über einen Atemschlauch und eine Gesichtsmaske während des Schlafes kontinuierlich zugeführt wird.

Wenn der Muskeltonus im Schlaf allgemein nachlässt, dann können die Weichteile des Atemweges im Rachenbereich kollabieren, und der Schläfer droht zu ersticken. Der entstandene Atemstillstand wird obstruktive Schlafapnoe genannt. Die Ursache des Kollabierens der weichen Atemwege ist ein Druckabfall in denselben, der durch die hohe Geschwindigkeit der Atemluftströmung verursacht wird. In der Folge können die Atemwege im Bereich Zungengrund und weicher Gaumen dem Druckunterschied zur äußeren normalen Umgebungsatmosphäre nicht mehr standhalten. Sie legen sich aneinander und verschließen den Luftweg. Die weiterhin wirkende Saugkraft der Lunge intensiviert den Verschluss noch, so dass die Patienten mehrere zehn Sekunden lang keine Luft mehr einatmen können. Der Vorgang kann in jeder Nacht während des Schlafes einige hundert Male auftreten. Die Langzeitfolgen sind eine Verminderung der Lebensqualität, Erkrankung des Herz-Kreislaufsystems und allgemein eine sinkende Lebenserwartung.

Wenn ein Patient mit einer obstruktiven Schlafapnoe aus einer künstlichen Atmosphäre atmet, deren Druck mindestens um den Betrag des strömungsbedingten Druckabfalls im Rachenbereich größer ist, als der Druck der natürlichen UmgebungsAtmosphäre, dann kann das weiche Gewebe im Rachenbereich nicht mehr kollabieren. Dieser Patient kann wieder frei und spontan atmen, und die Anzahl der Schlafapnoen reduziert sich auf das Maß eines gesunden Menschen. Die künstliche Atmosphäre wird hierzu von einem Beatmungsgerät erzeugt und dem Patienten über einen Atemschlauch und eine Atemmaske zugeführt.

Beatmungsgeräte, die eine künstliche Atmosphäre mit einem stets konstanten Therapiedruck erzeugen, sind unter der Bezeichnung CPAP-Geräte (CPAP = Continuous Positive Airway Pressure) bekannt.

Die Luftströmung in den Atemwegen eines Patienten erfolgt nicht widerstandsfrei. Auf die Länge verteilte laminare und turbulente Widerstandskomponenten wirken behindernd. Widerstandskomponenten sind Strömungwiderstände der Nasengänge, der weichwandigen Atemwege im Rachen und des Bronchialsystems. Die Größe eines turbulenten Widerstandes ist von der Strömungsstärke abhängig.

Die Atemwegswiderstände eines Patienten und die Strömungswiderstände eines Gerätes sind in Serie angeordnet. Der Innenwiderstand eines Gerätes kann durch eine entsprechende Gerätesteuerung beeinflusst werden. Eine geringe Belastung der Atemmuskulatur wird erzielt, wenn der Strömungswiderstand des Gerätes negativ ausgeführt ist und dadurch der Wirkung der positiven Atemwegswiderstände des Patienten entgegenwirkt. Ein negativer Widerstand besitzt ein umgekehrtes Druck-Strömungsverhalten. Auf den inneren Strömungswiderstand eines CPAP-Gerätes bezogen müsste dieses dann einen höheren Druck erzeugen, wenn es mit einem Luftstrom in Richtung Patient belastet wird und es müsste einen geringeren Druck erzeugen, wenn der Patient Luft in Richtung Gerät ausatmet.

CPAP-Geräte die während der Inspiration einen höheren Therapiedruck und während der Exspiration einen niedrigeren Therapiedruck erzeugen, sind als Bi-Level-Geräte bekannt (z.B. ein Gerät mit Namen BIPAP® der Firma RESPIRONICS). Zur Feststellung des Übergangs von einer Inspiration in eine Exspiration und umgekehrt besitzen diese Geräte einen Prozessor, der Atemtrigger genannt wird. Bi-Level-Geräte werden immer dann verwendet, wenn der Patient infolge anderer Krankheiten bereits einen allgemein erhöhten Atemwegswiderstand besitzt oder wenn eine Therapie mit einem CPAP-Gerät nur mit sehr hohem Therapiedruck erfolgreich wäre.

Die Steuerung des Druckes von Bi-Level-Geräten ist mit der Drucksteuerung von Beatmungsgeräten zur künstlichen Beatmung vergleichbar. Ein Nachteil besteht darin, dass nur zwei Druckniveaus existieren, die unabhängig von der jeweiligen Atemtiefe ohne eine Möglichkeit der Anpassung fest vorgegeben sind. Die durch den Atemtrigger veranlasste Druckumschaltung bewirkt einen rechteckförmigen Beatmungsdruckverlauf und somit keine optimale Kompensation der Widerstände der Atemwege. So bewirkt der Übergang vom kleineren zum höheren Druckniveau kurzzeitig eine künstliche Beatmung, indem Luft in die Lunge hineingedrückt wird. Beim Übergang vom hohen zum niederen Druckniveau kann Aufgrund der zu Beginn hohen Exspirationsströmung eine Einschnürung der kleinen Atemwege erfolgen und die Ausatmung sogar erschweren. Diese Erscheinungen sind unangenehm für den Patienten und können dadurch abgemildert werden, dass man den Übergang von dem einen auf das andere Druckniveau nicht exakt rechteckig gestaltet, sondern abrundet.

Der Komfort eines Bi-Level Gerätes wird entscheidend von der korrekten Funktion des Atemtriggers beeinflusst. Gerade am Ende der Exspirationsphase, wenn der Atemluftstrom fast zum Erliegen kommt, entstehen zusätzliche Luftschwingungen, weil das Herzschlagvolumen das Volumen der komprimierten Lunge und der daraus austretenden Luftströmung rhythmisch beeinflusst. Ein Atemtrigger kann deshalb das Ende einer Exspiration nicht genau erkennen und der Patient hat das Gefühl, dass die Druckumschaltung oftmals zum falschen Zeitpunkt erfolgt.

Aufgabe der Erfindung ist es daher, ein Beatmungsgerät zur Therapie von Schlafapnoe anzugeben, das in der Anwendung die Belastung der Atemmuskulatur des Patienten erheblich reduziert, den Behandlungsverlauf angenehmer macht und eventuelle Spätfolgen der Behandlung reduziert. In einem weiteren Aspekt der Erfindung soll ein Verfahren zur Steuerung eines solchen Gerätes angegeben werden.

Diese Aufgabe wird mit einem Beatmungsgerät mit den Merkmalen des Patentanspruchs 1 gelöst. Vorteilhafte Weiterbildungen eines solchen Beatmungsgerätes sind in den abhängigen Ansprüchen 2 bis 5 angegeben.

Eine weitere Lösung der Aufgabe besteht in einem Verfahren zur Steuerung eines solchen Beatmungsgerätes, wie es in Patentanspruch 6 genannt ist mit vorteilhaften Weiterbildungen gemäß den Ansprüchen 7 und 8.

Das erfindungsgemäße Beatmungsgerät enthält einen Druckerzeuger der einen bis zu einem frei wählbaren Grenzwert beliebigen Druck bereitstellen kann und von einer Druckregeleinrichtung gesteuert wird. Mit dem Druckerzeuger, z.B. einem Gebläse, erzeugt das Beatmungsgerät eine künstliche Atmosphäre, die dem Patienten mit Hilfe des Atemschlauches und der Atemmaske zugeführt wird.

Die erfindungsgemäß in dem Beatmungsgerät enthaltene Steuerung, die in einer vorteilhaften Weiterbildung ein Servoprozessor sein kann (vergleiche Anspruch 3), ermittelt einen Verlustdruck über dem Atemwegswiderstand und passt den Therapiedruckverlauf im wesentlichen proportional an den Verlauf des ermittelten Verlustdruckes an.

In einer Vorteilhaften Weiterbildung ist die Steuerung dazu eingerichtet, den Verlustdruck über dem Atemwegswiderstand aus den Größen von Atemluftstrom und Atemwegswiderstand des Patienten zu berechnen. Das Beatmungsgerät besitzt hierfür eine Vorrichtung zur Bestimmung des Atemluftstroms. Der Atemwegswiderstand des Patienten kann extern bestimmt und dem Beatmungsgerät in Form einer Eingabe mitgeteilt werden, oder dieser Widerstand wird, wie in einer vorteilhaften Weiterbildung gemäß Anspruch 4 angegeben, mit Hilfe einer im Beatmungsgerät integrierten Messvorrichtung automatisch permanent oder intermittierend gemessen.

Eine als vorteilhafte Weiterbildung mögliche kontinuierliche oder intermittierende Messung des Atemwegwiderstandes kann beispielsweise mit Hilfe eines aufgeprägten Druck- oder Luftstromsignals erfolgen. Dadurch werden Veränderungen des Atemwegwiderstandes bei der erfindungsgemäßen Steuerung des Beatmungsgerätes immer sofort berücksichtigt. Da die Zuführung der Luft aus dem Beatmungsgerät in der Regel über eine Nasenmaske erfolgt und der Strömungswiderstand der Nase recht großen Änderungen unterliegt, ist die kontinuierliche Messung des Atemwegwiderstandes insbesondere ein Weg, die unterschiedlich große Behinderung der Nasenatmung zu kompensieren.

Eine weitere vorteilhafte Ausgestaltung des erfindungsgemäßen Beatmungsgerätes ist die Kombination der neuartigen Eigenschaften mit der an sich hinlänglich bekannten und in der Patentliteratur z.B. in der EP 0705 615 A1 beschriebenen Arbeitsweise der nach dem Auto-Adjust Prinzip (Auto-CPAP) zur Ermittlung eines optimalen Basisdrucks arbeitenden Geräte (vgl. Anspruch 4). Ein erfindungsgemäß mit dieser Kombination ausgerüstetes Beatmungsgerät ermittelt dann automatisch den günstigsten Basisdruck, der sich ebenfalls mit der körperlichen Konstitution des Patienten ändern kann.

Unter Verzicht auf die oben beschriebenen vorteilhaften Ausgestaltungen können sowohl der Basisdruck als auch der Atemwegswiderstand mit externen Mitteln festgestellt und in das erfindungsgemäße Beatmungsgerät fest eingegeben werden.

Die mit dem erfindungsgemäßen Beatmungsgerät allgemein erzielte Wirkung ist die eines Atemkraftverstärkers, der eine Inspirationsanstrengung mit einer angepassten Druckerhöhung und eine Exspirationsanstrengung mit einer ebenso angepassten Druckabsenkung unterstützt. Vergleichbar ist diese Wirkung mit der eines Lenkkraftverstärkers oder eines Bremskraftverstärkers in einem Automobil.

Ein angenehmes Atmen wird dadurch erreicht, dass sowohl die Druckerhöhung während der Inspiration als auch die Druckabsenkung während der Exspiration nahezu proportional zum Verlustdruck am Atemwegswiderstand erfolgen. Zu diesem Zweck wird bei bekanntem Atemwegswiderstand der Verlustdruck als Produkt von Atemwiderstand und einem Anteil des Quadrates des Atemluftstrom berechnet, weil ein turbulenter Widerstand linear mit der Strömungsgeschwindigkeit anwächst. Die Größe des Anteils bestimmt den Grad der Atemkraftverstärkung.

Ebenfalls vorteilig ist die Anpassung an die verschiedenen Schlafstadien, die sich bekanntlich durch unterschiedliche Atemparameter wie Atemfrequenz und Atemzugvolumen unterscheiden. Auf diese Weise wird bei geringer Atemintensität eine anteilige künstliche Beatmung vermieden, indem die Differenz zwischen maximalem Inspirationsdruck und minimalem Exspirationsdruck automatisch kleiner wird. Bei großer Atemintensität hingegen erhöht sich die Differenz zwischen maximalem Inspirationsdruck und minimalem Exspirationsdruck entsprechend.

Aus der Vorgabe einer Intensität kann mit Hilfe der Steuerung außerdem festgelegt werden, ob der gesamte Verlustdruck über dem Atemwegswiderstand oder nur ein Teil davon durch das Beatmungsgerät kompensiert werden soll (vgl. Anspruch 6). Zu diesem Zweck werden das Ausgangssignal der Steuerung und das vorgegebene Basisdrucksignal addiert und als Führungsgröße der Druckregeleinrichtung zugeführt (vgl. Anspruch 7).

Die Erfindung soll nachfolgend an Hand eines Ausführungsbeispiels näher erläutert werden. In der zugehörigen Zeichnung zeigen:
- Fig 1: ein Blockschaltbild eines Ausführungsbeispiels eines erfindungsgemäßen Beatmungsgeräts und
- Fig 2: eine zeitliche Darstellung der Signalverläufe des Atemluftstromes *V̇ᵥ*, des Verlustdruckes Δp, des Therapiedruckes p_{act} und des intrapulmunalen Druckes p_{L} für unterschiedlich hohe Anteile einer Berücksichtigung des Verlustdruckes Δp bei der Festlegung des Therapiedruckes p_{act}.

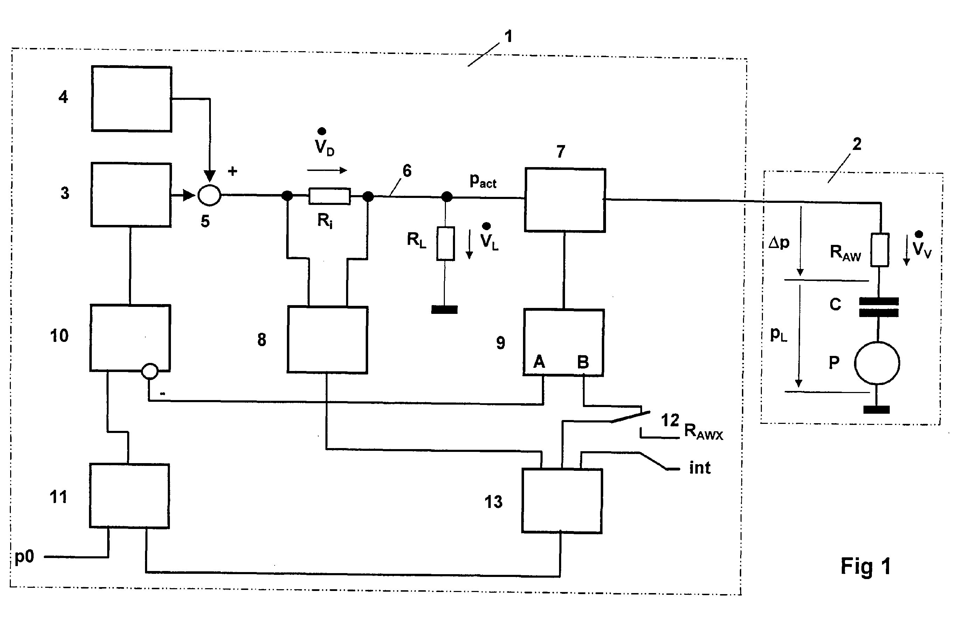

Das Blockschaltbild in der Figur 1 zeigt eine Variante, die eine mögliche Realisierung eines erfindungsgemäßen Beatmungsgerätes 1 darstellt.

Im Beatmungsgerät 1 befinden sich zur Druckerzeugung ein Druckerzeuger 3, der hier die Form eines Gebläses einnimmt, und eine Oszillationspumpe 4, die einen oszillierenden Druckverlauf mit kleiner Amplitude erzeugt. Beide Drücke werden an einer Summenstelle 5 zu einem Summendruck addiert und über einen Strömungswiderstand Rᵢ und einen Atemschlauch 6 in die Atemmaske 7 geleitet. Am Strömungswiderstand Rᵢ entsteht dadurch ein vom Geräteluftstrom *V̇_{D}* abhängiger Druckabfall. Ein Strömungsprozessor 8 ermittelt aus dem Signal des Geräteluftstrom *V̇_{D}* den Signalverlauf des Atemluftstroms *V̇ᵥ*, der die Lunge des Patienten 2 ventiliert. Das wird erreicht, indem der Verlauf des Leckageluftstrom *V̇_{L}* berechnet und vom Verlauf des Geräteluftstrom *V̇_{D}* subtrahiert wird. Ein Leckageluftstrom ist ein gewollter Luftstrom durch ein Ausatemventil R_{L}, das im Regelfall nur eine Öffnung ins Freie ist, und ein eventuell zusätzlicher parasitärer Luftaustritt durch eventuell undichte Teile der Maske.

Die Messung des Therapiedrucks p_{act} erfolgt in der Atemmaske 7, die normalerweise nur eine Nasenmaske ist. Der dort abgegriffene Druck wird einem Druckprozessor 9 zugeleitet, der zwei Ausgangssignale liefert. Das eine Signal am Ausgang A bildet den aktuellen Gleichdruckanteil ab und wird als Ist-Druck an die Druckregeleinrichtung 10 geleitet. Diese vergleicht den Ist-Druck mit einem Soll-Druck, der vom Addierer 11 geliefert wird und als Komponente den vorgegebenen Basisdruck p₀ des Beatmungsgerätes 1 enthält. Die Differenz zwischen Soll-Druck und Ist-Druck regelt die Größe des vom Druckerzeuger 3 zu erzeugenden Druckes in der Weise, dass möglichst keine bleibende Regelabweichung entsteht.

Das andere Signal am Ausgang B des Druckprozessors 9 bildet den Wechseldruck in der Atemmaske 7 ab. Dieser wird von der Oszillationspumpe 4 generiert. Seine Amplitude ist jedoch von den Größenverhältnissen aller vorhandenen Widerstände, dem Strömungswiderstand Ri, dem Atemwegswiderstand R_{AW} des Patienten 2 und dem Strömungswiderstand R_{L} einer Ausatemöffnung abhängig. Die Figur bezeichnet zum besseren Verständnis die Compliance C und die Atempumpe P des Patienten, die zur erfindungsgemäßen Funktion des Beatmungsgerätes 1 jedoch keinen Beitrag leisten.

Die Größen von Strömungswiderstand Rᵢ und dem Strömungswiderstand R_{L} der Ausatemöffnung sind bekannt, so dass der Druckprozessor den Wechseldruck über dem Atemwegswiderstand R_{AW} berechnen kann, der ein Maß für die Größe dieses Widerstandes ist.

Das Messergebnis wird über den Umschalter 12 einem Servoprozessor 13 zugeleitet, der durch Einbeziehung des festgestellten Atemluftstrom *V̇ᵥ* den Verlustdruck Δp über dem Atemwegswiderstand R_{AW} berechnet und aus der Vorgabe einer Intensität int bewertet ob der gesamte Verlustdruck Δp, der über dem Atemwegswiderstand R_{AW} verbraucht wird, oder nur ein Teil davon durch das Beatmungsgerät 1 kompensiert werden soll.

Durch Betätigen eines Umschalters 12 kann der Widerstandseingang des Servoprozessors 13 auf manuelle Eingabe eines Widerstandswertes R_{AWX} umgeschaltet werden. In dieser Betriebsart kann ein extern ermittelter Atemwegswiderstand fest vorgegeben werden, was für bestimmte Anwendungen sinnvoll sein kann.

Das Ausgangssignal des Servoprozessor 13 und der vorgegebene Basisdruck p₀ werden mit Hilfe des Addierers 11 zum Soll-Therapiedruck p_{act} für die Verarbeitung durch die Druckregeleinrichtung 10 addiert.

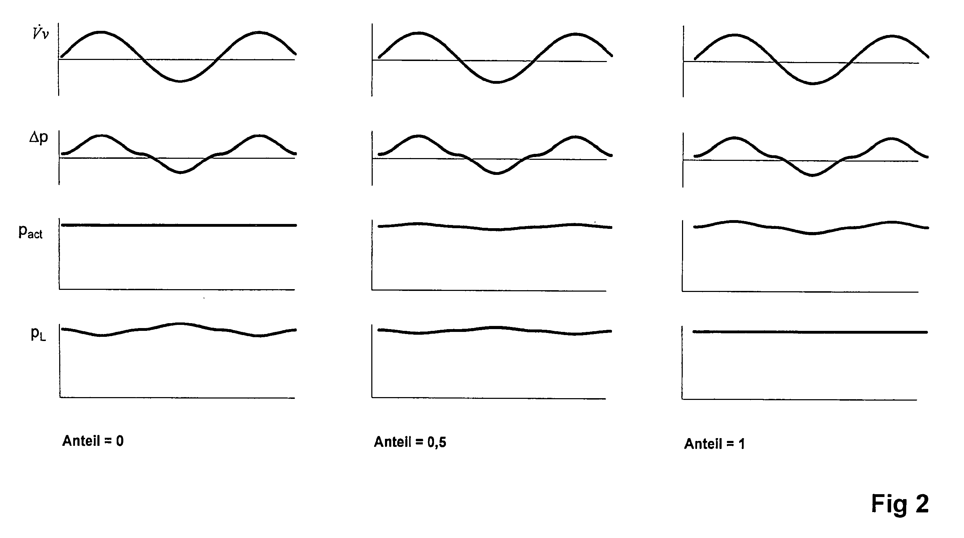

Die Figur 2 zeigt die Wirkungsweise des erfindungsgemäßen Beatmungsgerätes 1 im Signalzusammenhang. Zum leichteren Erkennen der Vorgänge werden diese schematisiert und es werden sinusförmige Erregungen betrachtet.

Sobald die Atempumpe P des Patienten 2 Inspirations- und Exspirationszyklen beschreibt, wird die Kapazität (Compliance) C der Lunge durch einen Atemluftstrom *V̇ᵥ* aufgeladen und entladen. Der Atemluftstrom *V̇ᵥ* fließt dabei durch den Atemwegswiderstand R_{AW} wodurch ein Teil des Therapiedrucks p_{act} verbraucht wird. Zwischen Druckverbrauch Δp und dem Luftstrom besteht kein linearer, sondern ein näherungsweise quadratischer Zusammenhang. Wie die Figur zeigt, besitzt der zeitliche Verlauf des Druckverbrauchs Δp deshalb keine sinusförmige Gestalt mehr.

Mit dem erfindungsgemäßen Beatmungsgerät wird der Therapiedruck p_{act} fortlaufend um einen Anteil des Verlustdrucks Δp verändert.

Wenn dieser Anteil Null ist (linke Darstellung in der Figur), dann ist der Therapiedruck p_{act} zeitlich unverändert. Der durch Inspiration und Exspiration hervorgerufene periodisch schwankende Verlauf des Verlustdrucks Δp bildet sich in diesem Fall vollständig auf Schwankungen des intrapulmonalen Drucks p_{L} ab. Für den Benutzer des Beatmungsgerätes bedeutet dies, dass er die auf seinen Atemwiderstand R_{AW} beruhenden Atembehinderungen ausnahmslos mit seiner Atemmuskulatur überwinden muss.

Mit höher werdendem Anteil (mittlere (Anteil=0,5) und rechte (Anteil=1) Darstellung in der Figur) wird der durch Inspiration und Exspiration hervorgerufene periodische Verlauf des Verlustdrucks Δp mehr und mehr auf Schwankungen des Therapiedrucks p_{act} verlegt. Folglich nehmen die Schwankungen des intrapulmonalen Drucks p_{L} in gleichem Maße ab. Das bedeutet für den Anwender, dass seine Atemanstrengungen kleiner werden. Die aus der Überwindung des Atemwegwiderstandes R_{AW} hergeleitete Atemarbeit wird mit steigendem Anteil vom Druckerzeuger übernommen. Selbst ein Anwender mit einem erhöhten Atemwegswiderstand R_{AW} benötigt dann für normale Inspirations- oder Exspirationsvorgänge keine großen Anstrengungen der Atemmuskulatur mehr. Er kann eine gleiche Ventilationsstärke mit einem viel geringeren intrapulmonalen Sog oder Druck realisieren.

Um die Größe des Atemwegwiderstandes R_{AW} ermitteln zu können, kann beispielsweise das Verfahren der oszillatorischen Atemwiderstandsmessung angewendet werden.

Eine andere Möglichkeit besteht in der Bestimmung des Atemwegwiderstandes durch externe Messverfahren und Eingabe des gemessenen oder anderweitig bestimmten Wertes.

Mit dem erfindungsgemäßen Beatmungsgerät können obstruktive Schlafapnoen behandelt werden, indem mit Hilfe des Druckerzeugers 3 eine künstliche Atmosphäre erzeugt wird, die dem Patienten 2 über den Atemschlauch 6 und die Atemmaske 7 zugeführt wird. Dabei verändert das Beatmungsgerät 1 den Druck der künstlichen Atmosphäre derart, dass der vorgegebene Therapiedruck p_{act} der künstlichen Atmosphäre um einen anteiligen Wert des an einem Atemwegswiderstand R_{AW} des Atemwegs des Patienten 2 verbrauchten Druckes Δp verändert wird. Bevorzugt wird der anteilige Wert, um den der Therapiedruck p_{act} der künstlichen Atmosphäre verändert wird für eine Inspiration oder eine Exspiration verschieden vorgegeben.

### Bezugszeichenliste

- 1: Beatmungsgerät
- 2: Patient
- 3: Druckerzeuger
- 4: Oszillationspumpe
- 5: Summenstelle
- 6: Atemschlauch
- 7: Atemmaske
- 8: Strömungsprozessor
- 9: Druckprozessor
- 10: Druckregeleinrichtung
- 11: Addierer
- 12: Umschalter
- 13: Servoprozessor

- A: Ausgang
- B: Ausgang
- C: Kapazität der Lunge (Compliance)
- int: Intensität
- P: Atempumpe
- P₀: Basisdruck
- p_{act}: Therapiedruck
- p_{L}: intrapulmonaler Druck
- R_{AW}: Atemwegswiderstand
- R_{AWX}: extern bestimmter Atemwegswiderstand
- Ri: Strömungswiderstand
- R_{L}: Strömungswiderstand
- *V̇_{D}*: Geräteluftstrom
- *V̇_{L}*: Leckageluftstrom
- *V̇ᵥ*: Atemluftstrom
- Δp: Verlustdruck

## Patentansprüche

1. Beatmungsgerät zur Behandlung von obstruktiver Schlafapnoe, mit einem Druckerzeuger (3) zum Erzeugen einer künstlichen Atmosphäre mit einem Therapiedruck (p_{act}), mit einer Druckregeleinrichtung (10) zum Führen des Druckerzeugers (3), und mit einem Atemschlauch (6) mit Atemmaske (7) zum Zuführen der von dem Druckerzeuger (3) erzeugten künstlichen Atmosphäre an einen Patienten (2), **gekennzeichnet durch** eine Steuerung (13) zum kontinuierlichen Ermitteln eines über einem Atemwegswiderstand (R_{AW}) der Atemwege des Patienten anfallenden Verlustdruckes (Δp) und zur im wesentlichen proportionalen Anpassung des Therapiedruckverlaufes an den Verlauf des ermittelten Verlustdruckes (Δp).

2. Beatmungsgerät nach Anspruch 1, **dadurch gekennzeichnet, dass** es eine Einrichtung (8) zum kontinuierlichen Bestimmen des aktuellen Atemluftstromes (*V̇*ᵥ) aufweist und dass die Steuerung (13) zum kontinuierlichen Ermitteln des Atemwegswiderstandes (R_{AW}) aus den Werten des Atemluftstromes (*V̇*ᵥ) und des Atemwegswiderstandes (R_{AW}) eingerichtet ist.

3. Beatmungsgerät nach einem der vorhergehenden Ansprüche, **dadurch gekennzeichnet, dass** die Steuerung (13) ein Servoprozessor ist.

4. Beatmungsgerät nach einem der vorhergehenden Ansprüche, **dadurch gekennzeichnet, dass** es ferner ein Einrichtung zum permanenten oder intermittierenden automatischen Bestimmen des Atemwegswiderstandes (R_{AW}) aufweist.

5. Beatmungsgerät nach einem der vorhergehenden Ansprüchen, **dadurch gekennzeichnet, dass** es eine nach dem Prinzip des Auto-CPAP arbeitende Einrichtung enthält, die einen optimalen Basisdruck (p₀) automatisch bestimmt.

6. Verfahren zur Steuerung eines Beatmungsgerätes gemäß einem der vorhergehenden Ansprüche, wobei der Druckerzeuger (3) zur Erzeugung des Therapiedruckes (p_{act}) von der Steuerung (13) so angesteuert wird, dass der Therapiedruck der künstlichen Atmosphäre ausgehend von einem vorgegebenen Basisdruck (p₀) durch Verändern um einen anteiligen Wert des aktuell ermittelten, an einem Atemwegswiderstand (R_{AW}) des Atemwegs des Patienten (2) verbrauchten Druckes (Δp) eingestellt wird.

7. Verfahren nach Anspruch 6, **dadurch gekennzeichnet, dass** der Steuerung (13) ein Intensitätssignal vorgegeben wird, das festgelegt, ob der gesamte über dem Atemwegswiderstand (R_{AW}) des Patienten verbrauchte Verlustdruck (Δp) oder nur ein Teil davon durch das Beatmungsgerät (1) kompensiert werden soll.

8. Verfahren nach Anspruch 7, **dadurch gekennzeichnet, dass** ein Ausgangswert der Steuerung (13) und ein Wert des vorgegebenen Basisdrucks (p₀) von einem Addierer (11) addiert werden und dass das erhaltene Summensignal der Druckregeleinrichtung (10) als Führungsgröße für einen durch den Druckerzeuger (3) einzuregelnden Therapiedruck (p_{act}) zugeleitet wird.
